# EUROPÄISCHE PATENTSCHRIFT

(11) **EP 1 703 865 B1**
(45) Veröffentlichungstag und Bekanntmachung des Hinweises auf die Patenterteilung: **24.02.2010**
(21) Anmeldenummer: 04762706.2
(22) Anmeldetag: 23.08.2004
(51) Int. Cl.: A61F 2/24, F16K 15/03

(54) **PROTHESE ZUM ERSATZ DER AORTEN- UND/ODER MITRALKLAPPE DES HERZENS**
PROTHESIS USED TO REPLACE THE AORTA AND/OR MITRAL VALVE OF THE HEART
PROTHESE DE REMPLACEMENT DE LA VALVULE AORTIQUE ET/OU DE LA VALVULE MITRALE DU COEUR

(30) Priorität: 29.08.2003 DE 10340265
(43) Veröffentlichungstag der Anmeldung: 27.09.2006
(73) Patentinhaber: Sievers, Hans-Hinrich, Prof. Dr. med., 24119 Kronshagen (DE)
(72) Erfinder: Sievers, Hans-Hinrich, Prof. Dr. med., 24119 Kronshagen (DE)
(74) Vertreter: Niedmers, Ole
(86) Internationale Anmeldenummer: PCT/DE2004/001866
(87) Internationale Veröffentlichungsnummer: WO 2005/023155

(56) Entgegenhaltungen:
- WO-A-91/07148
- US-A- 4 406 022
- US-A- 4 846 830
- US-B1- 6 395 024

## Beschreibung

Die Erfindung betrifft eine Prothese zum Ersatz der Aorten- und/oder Mitralklappe des Herzens, umfassend einen eine Mehrzahl von Klappenelementen gelenkig aufnehmenden Ringkörper, der in die Aorta und/oder in den Mitralklappenring einbringbar und dort mittels chirurgischer Maßnahmen befestigbar ist, wobei die Klappenelemente je nach Schwenkstellung den Durchlaß durch die Innenöffnung des Ringkörpers freigeben oder sperren.

Eine Prothese dieser Art ist bekannt (WO-A-85 04 094). Seit vielen Jahrzehnten werden Herzklappenprothesen bspw. in die vom Herzen wegführende Aorta implantiert, wobei die Herzklappenprothesen, von denen die ersten nach Art eines Kugel- oder Ballventils arbeitenden im Laufe der Zeit durch technisch immer ausgeklügeltere Konstruktionen ersetzt worden sind. Es sind Herzklappenprothesen mit einem Klappenelement, mit zwei Klappenelementen und auch mit drei Klappenelementen bekannt, bspw. die gattungsgemäße Prothese gem. der WO-A-85 04 094. Soweit es sich bei den Prothesen um solche handelt, die anstelle des kugel- bzw. ballförmig ausgebildeten Ventils Klappen bzw. Klappenelemente aufweisen, sind diese Klappenelemente am Ringkörper, der, um bei diesem Beispiel zu bleiben, in die Aorta einbringbar ist und dort mittels chirurgischer Maßnahmen, bspw. durch Vernähen, befestigbar sind, über Gelenke befestigt.

Dazu sind in der Regel am Ringkörper achsförmige Stege angeordnet, die in entsprechende Öffnungen des Klappenelements hineinragen und somit ein Gelenk für das Klappenelement bilden, wobei bei den Klappenelementen regelmäßig beidseitig derartige Öffnungen vorhanden sind, in die jeweils ein am Ringkörper ausgebildeter achsförmiger Steg hineinragt. Es sind im Stand der Technik aber auch Lösungen bekannt, bei denen an den Klappenelementen selbst achsartige Vorsprünge vorhanden sind, die in entsprechende im Ringkörper ausgebildete Öffnungen hineinragen.

Das eigentliche Öffnen und Schließen des Klappenelements bzw. der Klappenelemente, wenn mehrere Klappenelemente pro Ringkörper vorhanden sind, geht prinzipiell auch über lange Zeiträume des Einsatzes derartiger Prothesen mehr oder weniger störungsfrei vonstatten, da die Inhaltsstoffe des Blutes quasi als "Schmierstoff" wirken und somit die Reibung zwischen den Gelenkteilen reduzieren.

Die in der US6395024 B1 beschriebene Prothese bildet den Oberbegriff des Anspruchs 1.

Allen Herzprothesen der gattungsgemäßen Art haftet jedoch ein mehr oder weniger beim Einsatz fortwährend auftretender Nachteil an, daß nämlich die Gelenkbereiche zwischen Ringkörper und Klappenelementen die Strömungsdynamik des Blutes durch die Innenöffnung des Ringkörpers hindurch negativ beeinflussen, und zwar derart, daß sich in den Gelenkbereichen Zonen bilden, in denen das Blut nicht strömt. Zusätzlich wird dieser Effekt auch dadurch erzeugt, daß die Klappenelemente sich während des gesamten Herzzyklus nicht vollständig vom Ringkörper wegbewegen. In den Bereichen, in denen bei diesen Herzprothesen das Blut nicht strömt, bilden sich im Blut Gerinnsel, die sich von Zeit zu Zeit ablösen und mit dem durch die Aorta strömenden Blut auch in weit entfernte Körperteile transportiert werden, bspw. in das Gehirn, und dort die Blutbahnen verstopfen, mit den sich daraus ergebenden katastrophalen Folgen für den davon betroffenen Menschen.

Man hat im Gelenkbereich der Herzprothesen mit ausgeklügelten Konstruktionen versucht, die Gelenkbereiche, in denen quasi keine Strömung des Blutes stattfindet, so klein wie möglich zu halten, um die Gefahr der Gerinnselbildung zu minimieren und man hat auch versucht, die Gelenke bzw. die Gelenkbereiche zwischen den Klappenelementen und dem Ringkörper derart auszubilden, daß sie quasi durch das durch die Innenöffnung des Ringkörpers strömende Blut "ausgewaschen" bzw. "reingewaschen" werden, so daß dadurch die Gerinnselbildung ausgeschlossen werden sollte, alle diese Versuche haben aber keine wirklich befriedigende Lösung herbeigeführt, so daß nach wie vor auch immer noch auf Herzklappenprothesen, die zwei Klappenelemente aufweisen (sog. Zweiflügelklappen), oder die nach Art der Ball- bzw. Kugelventile, die keine Gelenke bzw. Gelenkverbindungen aufweisen, die aber auch erhebliche andere Nachteile aufweisen, zurückgegriffen worden ist und wird.

Es ist somit Aufgabe der vorliegenden Erfindung, eine Prothese der eingangs genannten Art zu schaffen, die die voraufgeführten Nachteile im Stand der Technik bekannter Prothesen, die Gelenke zwischen einem Klappenelement bzw. mehreren Klappenelementen und dem Ringkörper aufweist bzw. aufweisen, nicht hat, die konstruktiv in bezug auf ihre physikalisch-mechanische Wirkungsweise dem Aufbau der natürlichen Herzklappen nachempfunden sind, die beliebig lange im Körper nach der Implantation verbleiben können und eine gleichbleibende, störungsfreie Funktion gewährleisten und keinerlei Gefahr der Bildung von Blutgerinnsel zeitigen und auch ermöglichen, daß der sich während des Öffnens und des Schließens der Klappenelemente jeweils abfallende bzw. aufbauende Druckgradient des Blutes dem natürlichen Druckgradienten beim Öffnen und Schließen der natürlichen Herzklappen im wesentlichen entspricht. Eine weitere wesentliche Aufgabe der Erfindung ist es, eine Herzklappe der eingangs genannten Art zu schaffen, die nach ihrer Implantation keine fortwährende Verabreichung von blutgerinnungshemmenden Mitteln verlangt, so daß der Patient prinzipiell frei von einer fortwährenden Medikation nach der Implantierung der Herzklappen verbleiben kann.

Gelöst wird die Aufgabe gem. der Erfindung dadurch, daß die Gelenke der Klappenelemente ringseitig an in die Innenöffnung des Ringkörpers sich hineinerstreckenden Vorsprüngen im Bereich ihrer freien Enden ausgebildet sind.

Durch die erfindungsgemäß vorgenommene Maßnahme sind die bisherigen konstruktiven Prinzipien der Ausbildung der Gelenke zwischen dem Klappenelement bzw. den Klappenelementen und dem Ringkörper vollständig verlassen worden. Das ringseitige Gelenkteil, das mit dem klappenelementseitigen Gelenkteil das jeweilige Gelenk bildet, ist gezielt in Richtung des Zentrums bzw. wenn man so will, in Richtung einer gedachten Längsachse durch die Aorta hineinverschoben worden, um das jeweilige Gelenk soweit wie möglich in den Bereich der größten Strömungsgeschwindigkeit des Blutes durch die Innenöffnung des Ringkörpers hineinzuverlegen. Dadurch werden quasi alle Bereiche des jeweiligen Gelenks zwischen den Klappenelementen und dem Ringkörper fortwährend vom Blut, wo dessen Fließgeschwindigkeit am größten ist, umströmt, so daß sich keine strömungsfreien Bereiche in den Gelenkbereichen bilden können und somit eine Gerinnselbildung des Blutes an diesen Stellen quasi ausgeschlossen ist.

Ein weiterer außerordentlicher wesentlicher Vorteil, der mit der erfindungsgemäßen konstruktiven Lösung erreicht wird, ist der, daß damit ein verzögertes Schließen während der Vorwärtsströmung des Blutes (Systole) zeitig begonnen wird. Bei den bisher bekannten Prothesen geschieht dies überwiegend passiv durch das während der Erschlaffungsphase des Herzens zurückfließende Blut.

Gem. einer vorteilhaften Ausgestaltung der Prothese sind die Vorsprünge im Bereich ihrer vom Ringkörper abgewandten Enden mit einem Paar im wesentlichen in die Innenöffnung des Ringkörpers sich hineinerstreckenden, in Strömungsrichtung des Blutes durch den Ringkörper voneinander beabstandeten Stegen versehen, die jeweils ein ringseitiges Gelenk bilden. Das ringseitige Gelenk, das derart ausgebildet ist, kann somit fortwährend vom durch die Innenöffnung des Ringkörpers hindurchströmenden Blut umspült werden, wobei auch der Raum zwischen den beiden Stegen fortwährend durch das Blut durchströmt wird, da die beiden Stege derart beabstandet voneinander in die Strömungsbahn des Blutes hineinragend ausgebildet sind, daß der Zwischenraum von den Stegen störungsfrei durch das Blut durchströmt werden kann. Es sind somit am ringseitigen Teil des Gelenkes keine Bereiche vorhanden, in denen sich Blut sammeln kann, so daß keine Möglichkeit der Gerinnselbildung besteht.

Bei einer anderen vorteilhaften Ausführungsform kann wenigstens einer der Stege jeweils am Vorsprung benachbarter Klappenelemente integral ausgebildet sein, d.h., daß dadurch dort eine Kante bzw. Vertiefung weniger ausgebildet ist, in der sich Blut absetzen kann, wodurch die Strömung des Blutes den Gelenkbereich noch besser auswäscht.

Gem. einer weiteren vorteilhaften Ausgestaltung der Prothese werden die Gelenke der Klappenelemente klappenseitig lediglich durch zwei im wesentlichen symmetrisch zu einer gedachten Mittellinie der im wesentlichen flächig ausgebildeten Klappenelemente flächige Aussparungen gebildet, d.h. das klappenseitige Gelenkteil weist weder Vorsprünge noch Bohrungen oder Öffnungen oder dergl. auf, so daß sich auch an dem klappenseitigen Gelenkteil keine Bereiche ausbilden können, die vom strömenden Blut durch die Innenöffnung des Ringkörpers hindurch nicht erfaßt bzw. umspült werden.

Das ringseitige Gelenkteil und das klappenelementseitige Gelenkteil bilden vorteilhafterweise dadurch dank derart das Gelenk, daß das Klappenelement im Bereich der Aussparung zwischen den voneinander beabstandeten Stegen der beidseitig zu dem Klappenelement am Ringkörper korrespondierenden Vorsprünge gelenkig erfaßt wird. Dabei ist zwischen den beiden Stegen des ringseitigen Gelenkteils und dem zwischen diesen beiden Stegen jeweils ergriffenen Teil des Klappenelementes soviel Spiel, daß das durch die Innenöffnung des Ringelements strömende Blut fortwährend auch alle Bereiche der Gelenke durchströmen kann, d.h. Totzonen ausgeschlossen sind, in denen das Blut nicht strömt. Der Teil des Vorsprungs, in den das Klappenelement zwischen den Stegen hineinragt, ist vorzugsweise sphärisch bzw. kugelförmig ausgebildet, wodurch das Durchströmen des Blutes im Gelenkbereich keinem Strömungshindernis ausgesetzt ist.

Um zu verhindern, daß beim Schließen der Klappenelemente diese abrupt in ihre Schließstellung gehen, was aufgrund der dabei auftretenden hohen Druckgradienten zu Kavitationswirkungen führen kann und damit auch zu einer Schädigung, wenn nicht sogar Zerstörung des Blutes führen kann, kann die Prothese vorteilhafterweise dadurch weitergebildet werden, daß der herzzugewandte Steg des Stegpaares im wesentlichen orthogonal zur Strömungsrichtung des Blutes durch den Ringkörper verlaufend eine gekrümmte Gelenkfläche aufweist, auf der das Klappenelement bei seiner Öffnungs- und Schließbewegung abrollt. Mittels dieser Maßnahme wird ein gesteuerter Schließprozeß der Klappenelemente noch während der Strömung des Blutes in die Aorta hinein unterstützt, und zwar durch die durch diese konstruktive Maßnahme bewirkte Verminderung der Aufschlagkraft der Klappelemente beim Schließen auf den Ringkörper. Dadurch, daß auch durch diese Maßnahme extreme Druckgradienten auf das Blut vermieden werden, wird auch eine blutzerstörende kavitative Beeinflussung des Blutes während des Schließens der Klappenelemente vermieden.

Gem. einer weiteren vorteilhaften anderen Ausgestaltung der Prothese weist der herzabgewandte Steg des Stegpaares im wesentlichen orthogonal zur Strömungsrichtung des Blutes durch den Ringkörper verlaufend zwei Anschlagflächen auf, die jeweils die Öffnungsstellung und die Schließstellung des Klappenelementes definiert begrenzen. Durch diese Maßnahme wird vermieden, daß weitere Anschläge oder Begrenzungen, die in die Strömungsbahn des Blutes hineinragen können und somit Totzonen für die Strömung des Blutes bilden könnten, die auf alle Fälle vermieden werden müssen, nötig sind.

Vorteilhafterweise sind die Klappenelemente der Prothese sphärisch ausgebildet bzw. geformt, wobei die Sphäre in allen möglichen Freiheitsgraden ausgebildet sein kann. Eine so entstehende "bauchförmige" Vorwölbung der Klappenelemente beugt einem sogen. "Flow-Equilibrium" vor, d.h. die Klappenelemente können sich vollständig bis an den Anschlag anliegend während der Öffnungsphase öffnen und unterstützen damit nochmals das Umströmen bzw. Durchströmen der Gelenke, so daß auch durch diese Maßnahme zusätzlich einer Ablagerung und Gerinnselbildung von Blut ergänzend vorgebeugt werden kann. Die sphärische Ausbildung der Klappenelemente bewirkt auch die frühzeitige Einleitung des Schließens der Klappenelemente aufgrund der nachlassenden Strömung des Blutes auf die Klappenelemente in dieser Phase des Herzzyklus, d.h. schon während der Systole (Vorwärtsfluß). Dadurch ist für die Schließbewegung ein geringes Volumen an Blut für den Schließvorgang erforderlich mit der daraus sich für das Herz selbst ergebenden vorteilhaften Folge, daß das Herz weniger belastet wird.

Vorzugsweise ist der Teil des Klappenelementes zwischen einer gedachten Achsenlinie zwischen den Aussparungen und einer herzwärts gerichteten Spitze des Klappenelementes gegenüber dem auf der anderen Seite der Achslinie gelegenen anderen Teil des Klappenelementes gewinkelt ausgebildet, so daß bei maximaler Öffnung des Klappenelementes dieser Anteil parallel zur Strömungsrichtung des Blutes steht und dadurch eine Wirbelbildung verhindert wird. Dieser gewinkelte Teil kann auch sphärisch ausgebildet sein.

Aufgrund der Anordnung der Gelenke ringseitig an den freien Enden der Vorsprünge und klappenseitig aufgrund der in etwa seitenmittigen Aussparungen und aufgrund der sphärischen Ausbildung der Klappenelemente bewegen sich die Klappenelemente beim Öffnungsvorgang vollständig vom Ringkörper weg, was auch den Vorteil hat, daß dadurch alle Bereiche der Prothese im Inneren vom Blut umströmt werden.

Die erfindungsgemäße Prothese ist grundsätzlich gem. dem vorangehend beschriebenen Aufbau geeignet, lediglich mit einem Klappenelement oder zwei Klappenelementen, geeignet ausgebildet und geeignet im Ringkörper angeordnet, zu funktionieren. Es ist aber außerordentlich vorteilhaft, drei Klappenelemente in der Prothese vorzusehen, die, analog einem gleichseitigen Dreieck bzw. diesem angenähert, im Ringkörper gelenkig analog dem vorbeschriebenen konstruktiven Ausgestaltungen der Gelenke und der Stege ausgebildet sind. Auf diese sehr vorteilhafte Weise wird eine sogen. "3-Flügel-Klappe" mit der Prothese gem. der Erfindung geschaffen, wobei die Prothese damit in eine größtmögliche Nähe zur Herzklappenanordnung des natürlichen Herzens gelangt.

Dieser Vorgabe folgend ist es außerordentlich vorteilhaft, die Prothese derart weiter- bzw. auszubilden, indem durch geeignete Anordnung und Größe der Klappenelemente im Ringkörper vier im wesentlichen gleichgroße Durchflußöffnungen in der Innenöffnung des Ringkörpers gebildet werden. Es können somit bei einer "3-Flügel-Klappe" vier gleichgroße Durchflußöffnungen ausgebildet werden, wobei die so gebildete zentrale Durchflußöffnung und die drei durch den Ringkörper begrenzten randseitigen Durchflußöffnungen absolut ungestört von irgendwelchen Elementen das störungsfreie Durchströmen des Blutes gestatten.

Vorteilhaft ist es ebenfalls, die Prothese derart auszugestalten, daß sich die Innenöffnung des Ringkörpers von der zum Herzen gerichteten Einflußöffnung des Blutes durch die Innenöffnung des Ringkörpers hindurch zu seiner Ausflußöffnung im Querschnitt zunächst im wesentlichen bis zur Ebene der Vorsprünge verengend und im wesentlichen der Ebene der Vorsprünge nachfolgend stark gekrümmt erweiternd ausgebildet ist. Durch diese Maßnahme ist eine bestmögliche Anpassung an den Strömungsquerschnitt durch die Innenöffnung des Ringkörpers hindurch in Anlehnung an das natürliche Strömungsprofil im Bereich der natürlichen Herzklappen möglich.

Beim Eröffnen der Aorta während des Zeitpunktes der Implantation der erfindungsgemäßen Prothese ist aufgrund des fehlenden Innendruckes in der Aorta deren Durchmesser sehr viel kleiner als unter Druckbelastung. Aus diesem Grunde ist die erfindungsgemäße Prothese vorteilhafterweise derart gestaltet, daß sich der Außenquerschnitt des Ringkörpers von der Ausflußöffnung im wesentlichen bis zur Ebene der Vorsprünge vergrößert, so daß der Ringkörper mit seinem im Durchmesser kleineren Teil, d.h. dem Anteil des Ringkörpers, der unterhalb des Nahtrings liegt, beim Einsetzen in die Aorta eine Aufweitung, d.h. einen Dilatatoreffekt des natürlichen Implantationsringes, gewährleistet.

Um die Prothese mit dem Gewebe am Implantationsort dichtend befestigen zu können, weist der Ringkörper vorzugsweise an seiner Außenwandung im wesentlichen zwei voneinander beabstandete, nutartige, um den Ringkörper herumlaufende Vertiefungen auf, wobei in die zum Herzen gerichtete Vertiefung das verbleibende patienteneigene Gewebe eingebettet wird.

Vorzugsweise wird in die zweite Vertiefung, die dem Herzen weiter abgewandt ist als die vorgenannte erste Vertiefung, ein um den Ringkörper herumlaufender Nahtring angeordnet, mit dem das Gewebe im Zuge der Implantation der Prothese vernäht wird.

Als Werkstoff zur Ausbildung des Ringkörpers und/oder des Klappenelementes eignen sich letztlich alle Werkstoffe, die über die Zeit eine hohe Standfestigkeit bei geringem Gewicht haben und zudem in bezug auf biologisches Gewebe kompatibel sind. So können bspw. geeignete Metallegierungen aber auch elementare Metalle zur Ausbildung des Ringkörpers und/oder der Klappenelemente herangezogen werden. Es können aber auch Kunststoffwerkstoffe und auch Kunststoff-Verbundwerkstoffe zur Ausbildung des Ringkörpers und/ oder der Klappenelemente herangezogen werden.

Besonders vorteilhaft ist es, den Ringkörper und/oder die Klappenelemente aus Titan oder einer Titanlegierung herzustellen, wobei Titan bzw. eine Titanlegierung hochhart bei geringem Gewicht und hoher Verschleißfestigkeit sind, was insbesondere für den Bereich der Gelenke der erfindungsgemäßen Prothese von sehr großem Vorteil ist. Besonders vorteilhaft geeignet zur Ausbildung der Prothese sind Titan-Tantallegierungen.

Um die Verschleißfestigkeit der den Ringkörper und/oder die Klappenelemente bildenden Werkstoffe und deren Biokompatibilität zu erhöhen, ist es schließlich außerordentlich vorteilhaft, den Ringkörper und/oder die Klappenelemente mit einer Hartstoffschicht, bspw. aus Borcarbid oder dgl., zu versehen, bzw. zu beschichten, wobei diese mittels an sich bekannter klassischer Auftragverfahren wie dem PVD-Verfahren (Physical-Vapour-Deposition) und/oder CVP (Chemical-Vapour-Deposition) auf getragen werden können.

Die Erfindung wird nun anhand der Zeichnungen anhand eines Ausführungsbeispieles eingehend beschrieben. Darin zeigen:
- Fig. 1: in stark vergrößerter Darstellung eine Prothese gem. der Erfindung in der Draufsicht in geschlossener Stellung,
- Fig. 2: eine perspektivische Ansicht der Prothese gem. Fig. 1,
- Fig. 3: einen Schnitt entlang der Linie A-B von Fig. 1,
- Fig. 4: einen Schnitt entlang der Linie C-D von Fig. 3,
- Fig. 5: einen Schnitt entlang der Linie A-B von Fig. 1, wobei bei dieser Darstellung der Ringkörper der Prothese zwei um den Ringkörper außen herumlaufende Vertiefungen aufweist und wobei in einer Vertiefung ein Nahtring für die Verankerung der Prothese mit dem Gewebe angeordnet ist,
- Fig. 6: eine nochmals vergrößerte Darstellung des Details aus Fig. 3,
- Fig. 7: in stark vergrößerter Darstellung eine Prothese gem. der Erfindung in der Draufsicht in geöffneter Stellung,
- Fig. 8: eine perspektivische Darstellung der Prothese gem. Fig. 7,
- Fig. 9: einen Schnitt entlang der Linie E-F von Fig. 7,
- Fig. 10: eine nochmals vergrößerte Darstellung des Details von Fig. 9,

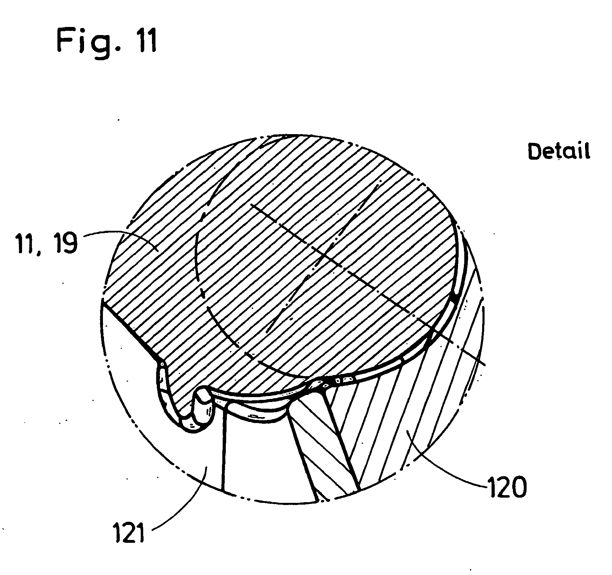

- Fig. 11: eine nochmals vergrößerte Darstellung des Details von Fig. 4,
- Fig. 12: in nochmals vergrößerter Darstellung ein Klappenelement in der Draufsicht,
- Fig. 13: eine Darstellung des Klappenelements gem. Fig. 12 in der Seitenansicht,
- Fig. 14: eine Darstellung des Kappenelements gem. Fig. 12 in der Seitenansicht, jedoch gegenüber der Darstellung von Fig. 13 mit sphärisch (bauchig) ausgebildeter Oberfläche,

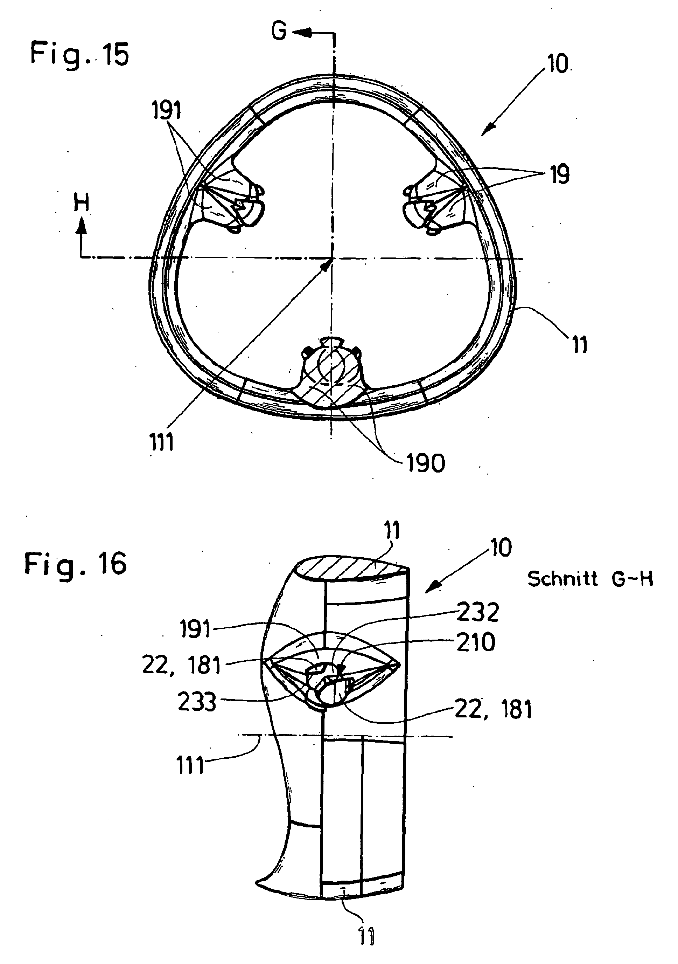

- Fig. 15: einen Ringkörper gem. der erfindungsgemäßen Prothese ohne Klappenelemente und einen in den Innenraum bzw. die Innenöffnung des Ringkörpers hineinstehenden Vorsprung im Schnitt zeigend, an dessen Ende das ringseitige Gelenkelement angeordnet ist.
- Fig. 16: einen Schnitt entlang der Linie G-H von Fig. 15 und

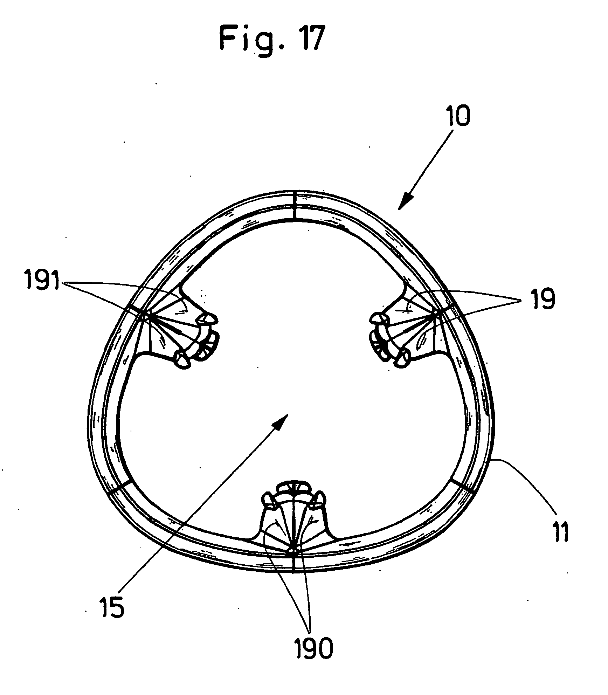

- Fig. 17: den Ringkörper der Prothese in vergrößerter Darstellung in der Draufsicht von hinten ohne Klappenelemente.

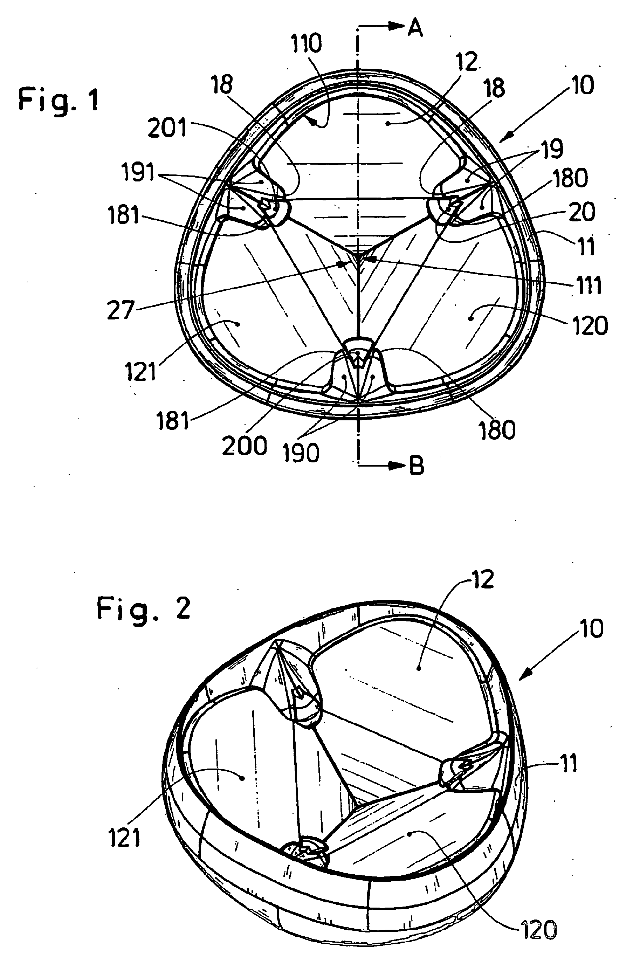

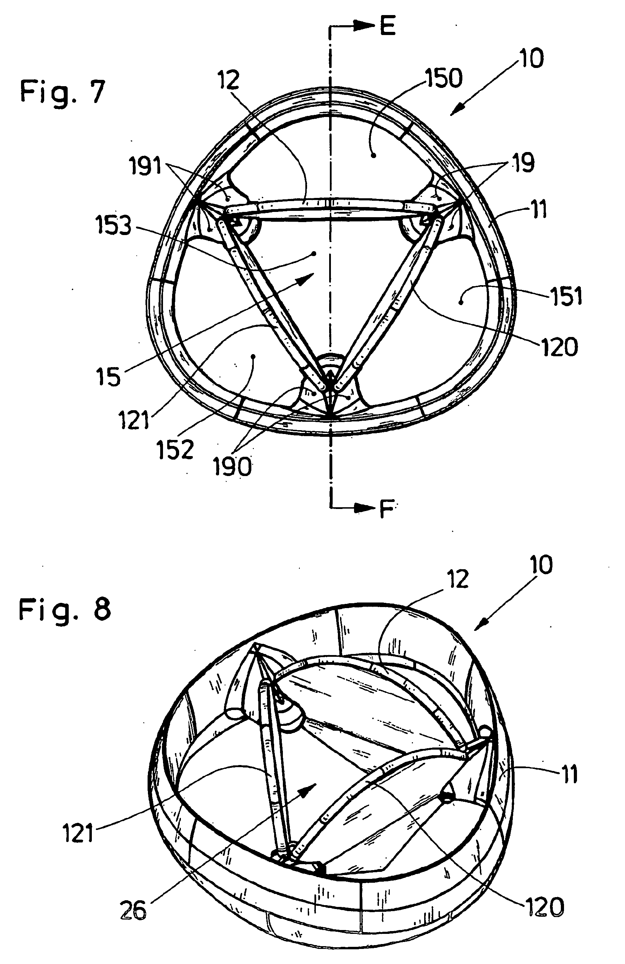

Es wird zunächst Bezug genommen auf die Darstellung der Prothese 10 gem. Fig. 1, die die Prothese 10 in Schließstellung zeigt, und auf die Darstellung gem. Fig. 7, die die Prothese 10 in geöffneter Stellung zeigt.

Die Figuren 1 und 7 zeigen die Prothese 10 gem. der Erfindung in der Draufsicht, und zwar von der Seite der Prothese 10, die nach der Implantation mit dem Herzen 14 verbunden wird bzw. auf das Herz 14 gerichtet ist.

Die Prothese 10 umfaßt im wesentlichen einen Ringkörper 11, der einen an ein gleichschenkliges Dreieck angenäherten Querschnitt aufweist, wobei die Ecken des Dreiecks allerdings nicht spitz auslaufen, sondern stark gerundet ausgebildet sind. Der Ringkörper 11 kann aber auch einen kreisförmigen oder beliebig geformten Querschnitt aufweisen. Die nachfolgend anhand eines Ausführungsbeispieles beschriebene Prothese 10 weist drei Klappenelemente 12, 120, 121 auf und wird nachfolgend auch immer im Zusammenhang mit drei Klappenelementen 12, 120, 121 beschrieben, das erfindungsgemäße Prinzip läßt sich aber auch grundsätzlich schon bei einer Prothese 10 realisieren, die lediglich mit einem Klappenelement oder zwei Klappenelementen versehen ist und grundsätzlich sind auch solche Konstruktionen denkbar, bei denen mehr als drei Klappenelemente 12, 120, 121 vorhanden sind. Der Ringkörper 11, der auf an sich bekannte Weise in die Aorta 13 und das Herz 14 einbringbar und dort mittels chirurgischer Maßnahmen befestigbar ist, nimmt die drei Klappenelemente 12, 120, 121 derart gelenkig auf, daß sie je nach Schwenkstellung der Klappenelemente 12, 120, 121 den Durchlaß des vom Herzen 14 gepumpten Blutes in die Aorta 13 durch die Innenöffnung 15 des Ringkörpers 11 freigegeben, vgl. Fig. 7, oder sperren, vgl. Fig. 1. Dieser Mechanismus ist als solcher bekannt und findet sich grundsätzlich bei allen Prothesen 10 dieser Art, so daß an dieser Stelle darauf nicht weiter eingegangen zu werden braucht, zumal die Fachwelt diesen Mechanismus grundsätzlich kennt.

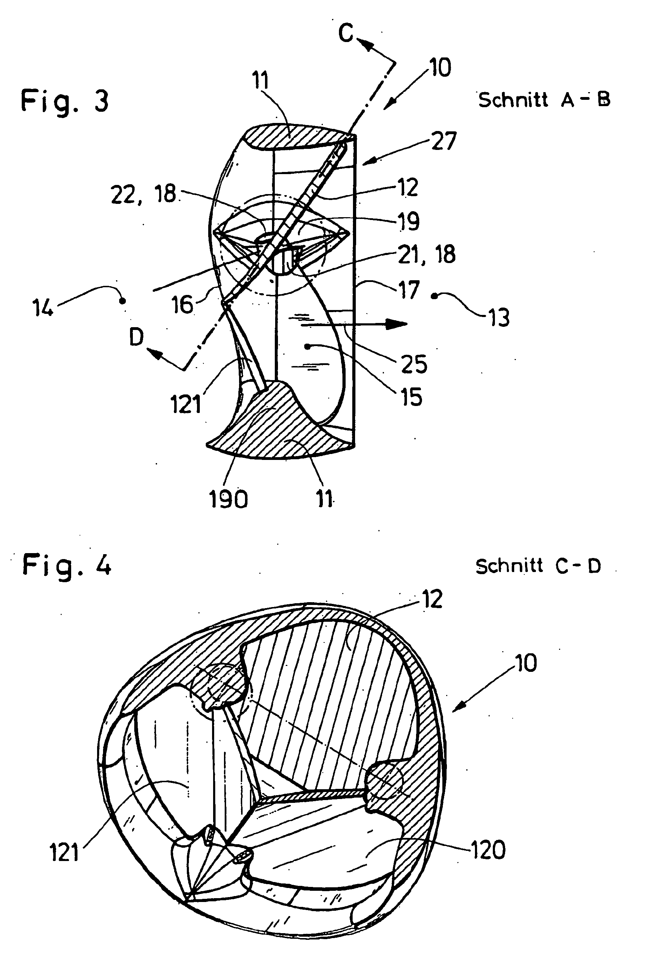

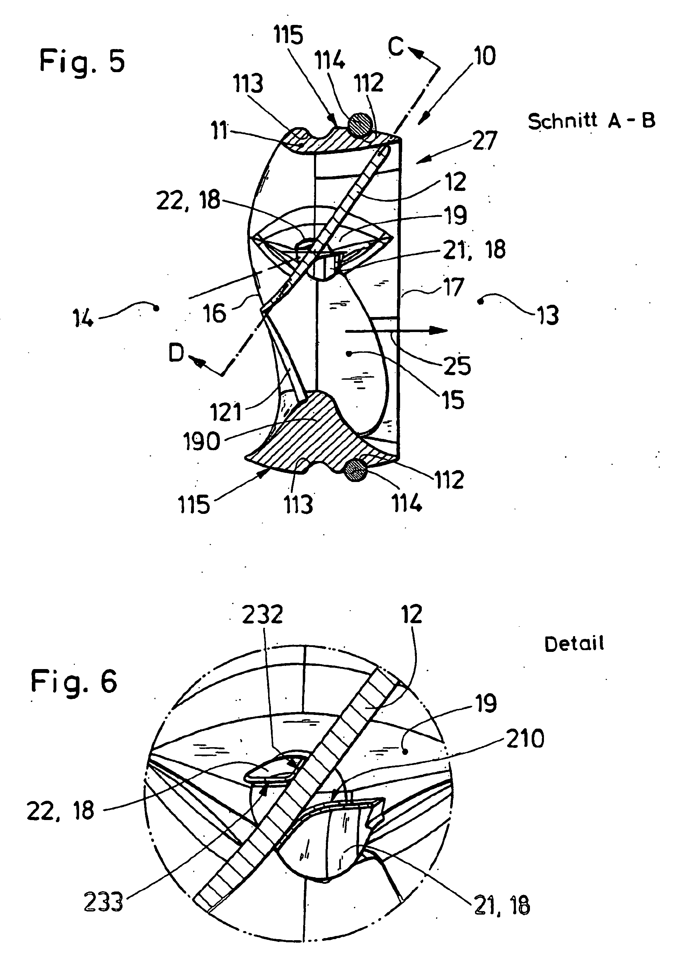

In den Innenraum bzw. die Innenöffnung 15 des Ringkörpers 11 stehen drei im wesentlichen identisch ausgestaltete und voneinander gleich im Ringkörper voneinander beabstandete Vorsprünge 19, 190, 191 hinein, die integral mit dem Ringkörper 11 ausgebildet werden können, wobei noch weiter unten darauf im einzelnen eingegangen wird. Die Vorsprünge 19, 190, 191 sind im Längs- und Querschnitt derart gestaltet, daß sie eine weitgehend laminare Strömung des Blutes durch die Innenöffnung 15 des Ringkörpers 11 gestatten, d.h. sie sind im Hinblick auf ihr Quer- und Längsprofil in bezug auf hydrodynamische Parameter derart profiliert ausgebildet, daß sie einen geringstmöglichen hydrodynamischen Widerstand dem durch die Innenöffnung 15 des Ringkörpers 11 fließenden Blut bieten, vgl. auch den in Fig. 3 dargestellten Schnitt entlang der Linie A-B von Fig. 1 sowie das in Fig. 6 dargestellte Detail von Fig. 3.

Die Vorsprünge 19, 190, 191 stehen beispielhaft, ausgehend vom Mittelpunkt bzw. einer gedachten Innenachse 111 durch den Ringkörper 11 in Strömungsrichtung 25 des Blutes hindurch bis zur Innenwandung 110, im Bereich von ca. 45 % der gesamten Länge zwischen Innenwandung 110 und der gedachten Längsachse 111 in den Innenraum bzw. die Innenöffnung 15 hinein. Es sei aber darauf hingewiesen, daß diese Angabe keine Begrenzung oder Festlegung der effektiven Länge der Vorsprünge 19, 190, 191 bedeutet, ausgehend von der Innenwandung 110 des Ringkörpers 11 in die Innenöffnung 15 hinein, denn grundsätzlich sind auch andere Längen der Vorsprünge 19, 190, 191 denkbar, die in Anpassung an die individuellen Konditionen des Patienten gewählt werden können, in den die Prothese 10 implantiert werden soll. Diese Längenangabe (im Bereich von 45 %) soll hier lediglich als Erläuterung dahingehend verstanden werden, daß bei der erfindungsgemäßen Prothese 10 bewußt die Vorsprünge relativ zur Gesamtlänge zwischen Innenwandung 110 und der gedachten Längsachse 111 derart groß gewählt wird, daß sich der Bereich der Vorsprungenden 20, 200, 201, soweit konstruktiv vertretbar, bis in den Bereich des kleinsten Strömungsgradienten des Blutes durch die Innenöffnung 15 des Ringkörpers 11 hindurch erstreckt.

Ringseitig sind die Klappenelemente 12, 120, 121 durch Gelenke 18, 180, 181 befestigt, die an den zur Längsachse 110 gerichteten Vorsprungsenden 20, 200, 201 ausgebildet sind. Die jeweiligen Gelenke 18, 180, 181 sind, bei der in den Figuren beschriebenen Ausgestaltung der Prothese 10, jeweils an allen Vorsprüngen 19, 190, 191 doppelt ausgebildet, so daß jeder der Vorsprünge 19, 190, 191 zwei Gelenke 18, 180, 181 trägt bzw. an ihnen ausgebildet sind, so daß, vgl. insbesondere die Figuren 1 und 7, pro Vorsprung 19, 190, 191 jeweils die einen Gelenke jeweils benachbarter Klappenelemente 12, 120, 121 ausgebildet werden. Das bedeutet, daß anstelle bisheriger Konstruktionen, um bei der Ausbildung von Prothesen 10 mit drei Klappenelementen zu bleiben, lediglich drei Vorsprünge mit Gelenken in die Innenöffnung 15 der Prothese 10 hineinragen und lediglich drei Vorsprünge die angestrebte laminare Strömung des Blutes durch den Ringkörper 11 hindurch stören können, wohingegen bei allen bisherigen Konstruktionen der Prothese 10 mit drei Klappenelementen sinngemäß sechs Gelenkaufnahmen bzw. Gelenkaufnahmeorte nötig waren, mit den sich daraus ergebenden, in bezug auf den hydrodynamischen Widerstand nachteiligen Folgen aufgrund einer damit naturgemäß verbundenen Vergrößerung des hydrodynamischen Widerstandes. Pro Vorsprung 19, 190, 191 werden die Gelenke 18, 180, 181 ringseitig durch jeweils zwei auf die Längsachse 111 im wesentlichen gerichtete, voneinander beabstandete Stege 21, 22 gebildet, die sich im wesentlichen in Strömungsrichtung 25 des Blutes erstrecken, vgl. Figuren 3, 6, 9 und 10. Die beiden Stege 21, 22 bilden als jeweiliges Paar jeweils gemeinsam ein ringseitiges Gelenkteil 18, 180, 181, wobei bei der hier beschriebenen Ausgestaltung der Prothese 10 mit den drei Klappenelementen 12, 120, 121 sechs Gelenke 18, 180, 181, bestehend aus jeweiligen Stegpaaren 21, 22, gebildet werden.

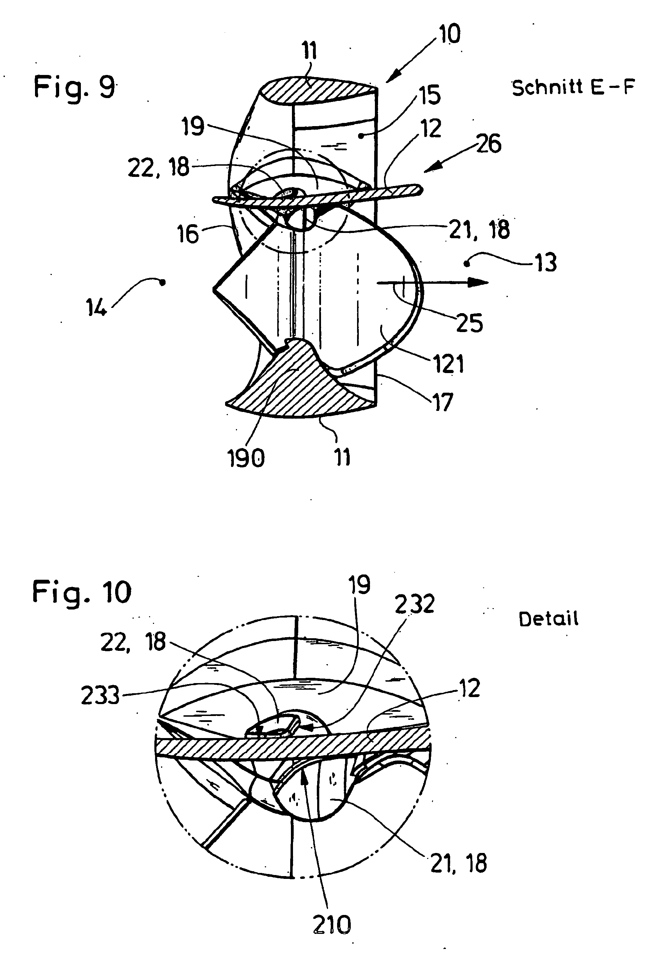

Der druckabgewandte Steg 21, d.h. der Steg 21, der der Aorta 13 zugewandt ist, ist im wesentlichen orthogonal zur Strömungsrichtung 25 des Blutes durch den Ringkörper 11 verlaufend ausgebildet. Dieser weist eine gekrümmte Gelenkfläche 210 auf. Diese gekrümmte Gelenkfläche 210 dient dazu, daß auf dieser das Klappenelement 12 , 120, 121 bei seiner Öffnungs- und Schließbewegung abrollen kann, vgl. auch die Fig. 3, das Detail gem. Fig. 6 sowie die Fig. 9 und das Detail gem. Fig. 10. Durch diese gekrümmte Gelenkfläche 210 ist gewissermaßen ein Abrollen im Zuge der Schwenkbewegung des jeweiligen Klappenelementes 12, 120, 121 möglich. Der druckzugewandte Steg 22 des Stegpaares ist ebenfalls im wesentlichen orthogonal zur Strömungsrichtung 25 des Blutes durch den Ringkörper 11 verlaufend ausgebindet. Der druckzugewandte Steg 22 weist anstelle der gekrümmten Gelenkfläche 210 des druckabgewandten Steges 21 zwei Anschlagflächen 232, 233 auf. Diese Anschlagflächen 232, 233 begrenzen jeweils die Öffnungsstellung 26, vgl. die Fig. 9 und das Detail gem. Fig. 10, sowie die Schließstellung 27 der Klappenelemente 12, 120, 121 definiert, vgl. die Fig. 3 sowie das Detail gem. Fig. 6.

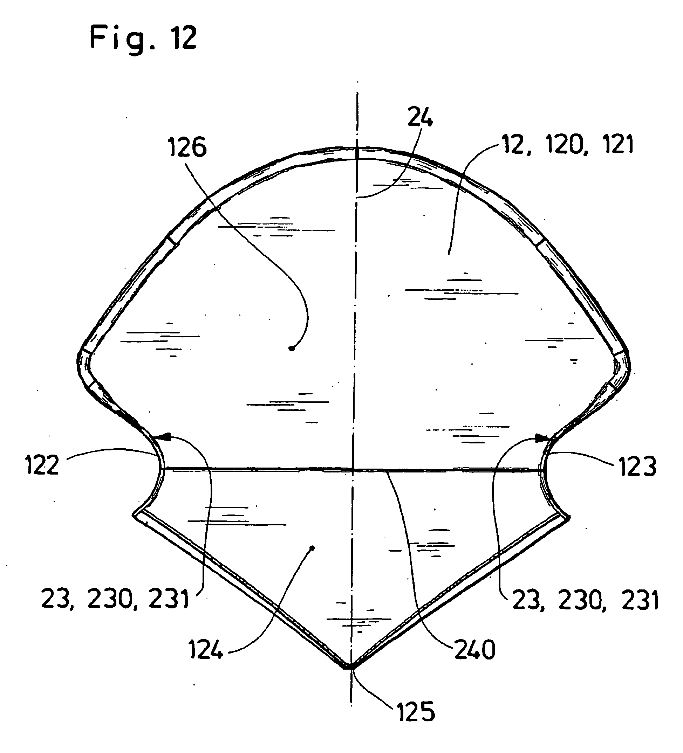

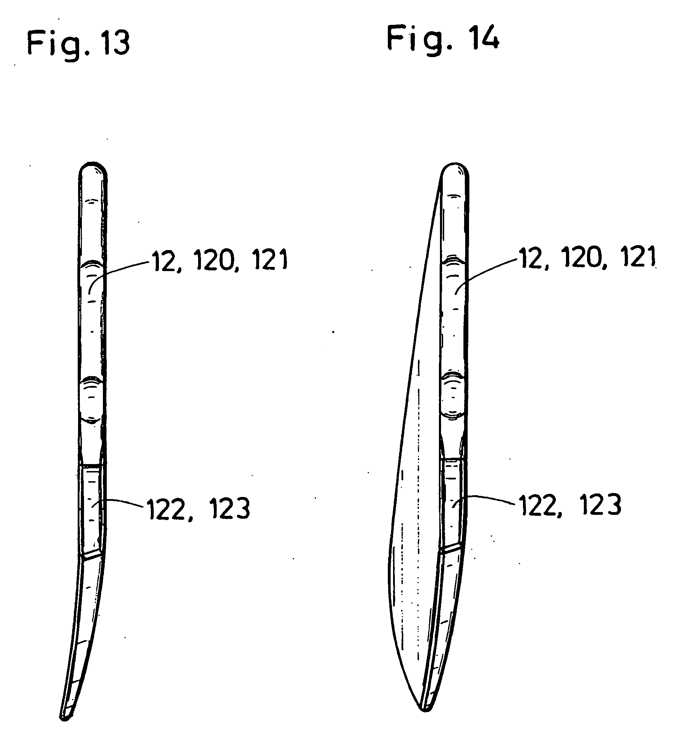

Die klappenseitigen Gelenke 23, 230, 231, d.h. jeweils zwei Gelenke 23, 230, 231 pro Klappenelement 12, 120, 121, werden durch zwei im wesentlichen symmetrisch zu einer gedachten Mittellinie 24, vgl. Fig. 12, vorgesehene flächige Aussparungen 122, 123 gebildet. Die Klappenelemente 12, 120, 121 sind zumindest in einem Freiheitsgrad gebogen, vgl. Fig. 13, ausgebildet, die Klappenelemente 12, 120, 121 können insgesamt aber auch sphärisch, d.h. "bauchig", ausgebildet sein, vergl. Fig. 14. Die "Gelenke" bzw. Gelenkteile 23, 230, 231 der Klappenelemente 12, 120, 121 sind somit lediglich Aussparungen 122, 123 in der Fläche der Klappenelemente 12, 120, 121. Mittels dieser sehr einfachen, aber in hohem Maße effektiven und äußerst funktionsoptimierten Form, bei denen keine Bohrungen oder Achsbolzen oder dgl. vorgesehen werden müssen, wird das jeweilige Gesamtgelenk zwischen den ringkörperseitigen Gelenken bzw. Gelenkteilen 18, 180, 181 und den klappenelementseitigen Gelenken bzw. Gelenkteilen 23, 230, 231 dadurch gebildet, daß das Klappenelement 12, 120, 121 jeweils im Bereich der Aussparung 122, 123 zwischen den voneinander beabstandeten Stegen 21, 22 beidseitig erfaßt wird.

Das Klappenelement 12; 120, 121 wird somit am Ringkörper beidseitig entsprechend der korrespondierenden Vorsprünge 19, 190, 191 über die jeweils dort ausgebildeten Stege 21, 22 gelenkartig erfaßt.

Somit werden, vgl. insbesondere die Figuren 7 und 8, in Öffnungsstellung 26 der Klappenelemente 12, 120, 121 vier für den Durchfluß des Blutes durch die Innenöffnung 15 des Ringkörpers 11 im wesentlichen gleichgroße Durchflußöffnungen 150, 151, 152, 153 gebildet. Durch geeignete Anordnung und Dimensionierung der Klappenelemente 12, 120, 121 im Ringkörper 11 können somit Durchflußöffnungen 150, 151, 152, 153 geschaffen werden, die alle gleichgroße Strömungsquerschnitte für das Blut aufweisen.

Die Innenöffnung 15, vgl. insbesondere die Figuren 3 und 9, die jeweilige Schnitte durch den Ringkörper 11 darstellen, ist derart gestaltet, daß sich die Innenöffnung 15 von der zur Aorta 13 gerichteten Ausflußöffnung 17 des Blutes aus zunächst verengt, und zwar im wesentlichen bis zur Ebene der Vorsprünge 19, 190, 191. Von dort aus in Strömungsrichtung 25 nachfolgend erweitert sich die Innenöffnung 15 bis zur Ebene der Einflußöffnung 16, und zwar auf den Querschnitt des Ringkörpers 11 bezogen, auf sehr starke Weise. Der Außenquerschnitt des Ringkörpers 11 vergrößert sich, von der Einflußöffnung 16 im wesentlichen bis zur Ebene der Vorsprünge 19, 190, 191, so daß quasi eine Dehnung der Aorta 13 bei der Implantation der Prothese 11 vonstatten gehen kann, da die Aorta 13 während der Implantation aufgrund des fehlenden Dehnungsdruckes auf die Innenwand des Blutes 14 im Durchmesser kleiner ist als unter Druckbelastung. Aufgrund der vorangehend beschriebenen konstruktiven Ausgestaltung des Außenquerschnittes des Ringkörpers 11 können prinzipiell größere Prothesen 10 implantiert werden und der Druckgradient kann gesenkt werden, so daß die erfindungsgemäße Prothese 10 nicht mehr als Druckhindernis und Strömungsverwirbelungsgegenstand wirkt.

Der Ringkörper 11 weist an seiner Außenwandung 115, vergl. Fig. 5, hier im Querschnitt halbrundförmig ausgebildete, um den Ringkörper 11 herumlaufende Vertiefungen 112, 113 auf. Die Vertiefungen 112, 113 sind auf der Außenwandung 115 des Ringkörpers 11 voneinander beabstandet ausgebildet. In der dem Herzen 14 zugewandten Vertiefung 112 kommt das verbleibende patientenseitige Gewebe zu liegen. In der dem Herzen 14 weiter abgewandten, um den Ringkörper 11 herumlaufenden Vertiefung 112 wird ein Nahtring 114 angeordnet, der ebenfalls in der Vertiefung 113 liegend um den Ringkörper 11 herumläuft. Dieser Nahtring 114 liegt hermetisch fest in der Vertiefung 113. Der Nahtring 114 kann aus geeignetem Kunststoffwerkstoff bestehen und dient der Befestigung des Gewebes der Aorta 13 durch geeignetes Vernähen.

Der Ringkörper 11 kann, wie eingangs schon angedeutet, als einstückiges Teil, als Spritzgußteil oder mittels pulvermetallurgischer Vorfahren hergestellt werden, was gleichermaßen auch für die Klappenelemente gilt. Der Ringkörper 11 und/oder die Klappenelemente 12, 120, 121 können vorzugsweise aus Titan oder Titanlegierungen bestehen, wobei die Herstellung auf vorbeschriebene Weise erfolgen kann. Der Ringkörper 11 und/oder die Klappenelemente 12, 120, 121 können auch mit einer Hartstoffschicht versehen sein, um eine nekrotische Wirkung bestimmter Metall-Legierungen, die zur Ausbildung der Prothese 10 insgesamt verwendet werden, zu vermeiden und eine Biokompatibilität in bezug auf biologisches Gewebe zu gewährleisten und können auch mit einer hochharten und verschleißfesten Schicht versehen werden, was insbesondere für den Bereich der Gelenke 23, 18, 230, 180 und 231, 181 zwischen Ringkörper 11 und Klappenelementen 12, 120, 121 gilt. Durch Aufbringen dieser Schicht findet kein meßbarer Verschleiß zwischen den Gelenken der Prothese 10 mehr statt.

Grundsätzlich ist es auch möglich, den Ringkörper 11 mehrteilig, bspw. dreiteilig, auszubilden, und nach dem Montieren der Klappenelemente 12, 120, 121 in den Gelenken 18, 23, 180, 230, 181, 231 auf geeignete Weise zusammenzufügen.

Bei einer einstückigen Ausbildung des Ringkörpers 11 können die Klappenelemente 12, 120, 121 durch geringfügige elastische Verformung zwischen die jeweiligen Stege 21, 22 der Vorsprünge 19, 190, 191 über ihre Aussparungen 122, 123 "eingeklippt" werden.

In der Beschreibung ist die Prothese 10 gem. der Erfindung im wesentlichen im Zusammenhang mit ihrer Implantierung in die Aorta 13 beschrieben worden. Die erfindungsgemäße Prothese 10 ist jedoch auch bei prinzipiell gleichem Aufbau wie vorangehend beschrieben als Mitralklappe verwendbar bzw. implantierbar.

### Bezugszeichenliste

| | | | |
|---|---|---|---|
| 10 | Prothese | 191 | Vorsprung |
| 11 | Ringkörper | 20 | Vorsprungende |
| 110 | Innenwandung | 200 | Vorsprungende |
| 111 | Längsachse | 201 | Vorsprungende |
| 112 | umlaufende Vertiefung | 21 | Steg (aortazugewandt) |
| 113 | umlaufende Vertiefung | 210 | gekrümmte Gelenkfläche |
| 114 | Nahtring | 22 | Steg (aortaabgewandt) |
| 115 | Außenwandung | 23 | Gelenk |
| 12 | Klappenelement | 230 | Gelenk |
| 120 | Klappenelement | 231 | Gelenk |
| 121 | Klappenelement | 232 | Anschlagfläche |
| 122 | Aussparung | 233 | Anschlagfläche |
| 123 | Aussparung | 24 | Mittellinie (Klappenelement |
| 124 | Teil des Klappenelementes | 240 | Achsenlinie (Klappenelement) |
| 125 | herzwärts gerichtete Spitze des Klappenelementes | 25 | Strömungsrichtung (Blut) |
| 126 | Teil des Klappenelementes | 26 | Öffnungsstellung |
| 13 | Aorta /aortaseitig | 27 | Schließstellung |
| 14 | Herz /herzseitig | | |
| 15 | Innenöffnung (Ringkörper) | | |
| 150 | Durchlaßöffnung | | |
| 151 | Durchlaßöffnung | | |
| 152 | Durchlaßöffnung | | |
| 153 | Durchlaßöffnung | | |
| 16 | Einflußöffnung (Ringkörper) | | |
| 17 | Ausflußöffnung (Ringkörper) | | |
| 18 | Gelenk | | |
| 180 | Gelenk | | |
| 181 | Gelenk | | |
| 19 | Vorsprung | | |
| 190 | Vorsprung | | |

## Patentansprüche

1. Prothese (10) zum Ersatz der Aorten- und Mitralklappe des Herzens (14), umfassend einen eine Mehrzahl von Klappenelemente (12, 120, 121) gelenkig aufnehmenden Ringkörper (11), der in die Aorta (13) und/oder den Mitralklappenring einbringbar und dort mittels chirurgischer Maßnahmen befestigbar ist, wobei die Klappenelemente (12, 120, 121) je nach Schwenkstellung den Durchlaß durch die Innenöffnung (15) des Ringkörpers (11) freigeben oder sperren, **dadurch gekennzeichnet, daß** die Gelenke (18, 180, 181) der Klappenelemente (12, 120, 121) ringseitig an in die Innenöffnung (15) des Ringkörpers (11) sich hineinerstreckenden Vorsprüngen (19, 190, 191) ausgebildet sind.

2. Prothese nach Anspruch 1, **dadurch gekennzeichnet, daß** an den Vorsprüngen (19; 190; 191) im Bereich ihrer vom Ringkörper (11) abgewandten Enden (20; 200; 201) wenigstens ein Paar im wesentlichen in die Innenöffnung (15) des Ringkörpers (11) sich hineinerstreckende, in Strömungsrichtung (25) des Blutes durch den Ringkörper (11) voneinander beabstandete Stege (21, 22) ausgebildet sind, die jeweils ein ringseitiges Gelenk (23, 230, 231) bilden.

3. Prothese nach einem oder beiden der Ansprüche 1 oder 2, **dadurch gekennzeichnet, daß** die Gelenke (23; 230; 231) der Klappenelemente (12; 120; 121) klappenseitig durch zwei im wesentlichen symmetrisch zu einer gedachten Mittellinie (24) der im wesentlichen flächig ausgebildeten Klappenelemente (12; 120; 121) flächige Aussparungen (122, 123) gebildet werden.

4. Prothese nach einem oder mehreren der Ansprüche 1 bis 3, **dadurch gekennzeichnet, daß** wenigstens einer der Stege (21; 22) für jeweils an einem Vorsprung (19; 190; 191) benachbarter Klappenelemente (12; 120; 121) integral ausgebildet sind.

5. Prothese nach einem oder beiden der Ansprüche 3 oder 4, **dadurch gekennzeichnet, daß** das Klappenelement (12; 120; 121) im Bereich der Aussparungen (122, 123) zwischen den voneinander beabstandeten Stegen (21, 22) der beidseitig zudem Klappenelement (12; 120; 121) am Ringkörper (11) korrespondierenden Vorsprünge (19; 190; 191) gelenkig erfaßt wird.

6. Prothese nach einem oder mehreren der Ansprüche 2 bis 5, **dadurch gekennzeichnet, daß** der aortazugewandte Steg (21) des Stegpaares im wesentlichen orthogonal zur Strömungsrichtung (25) des Blutes durch den Ringkörper (11) verlaufend eine gekrümmte Gelenkfläche (210) aufweist, auf der das Klappenelement (12; 120; 121) bei seiner Öffnungs- und Schließbewegung abrollt.

7. Prothese nach einem oder mehreren der Ansprüche 1 bis 6, **dadurch gekennzeichnet, daß** der aortaabgewandte Steg (22) des Stegpaares im wesentlichen orthogonal zur Strömungsrichtung (25) des Blutes durch den Ringkörper (11) verlaufend zwei Anschlagflächen (232; 233) aufweist, die jeweils die Öffnungsstellung (26) und die Schließstellung (27) des Klappenelements (12) definiert begrenzen.

8. Prothese nach einem oder mehreren der Ansprüche 1 bis 7, **dadurch gekennzeichnet, daß** das Klappenelement (12; 120; 121) sphärisch ausgebildet ist.

9. Prothese nach einem oder mehreren der Ansprüche 1 bis 8, **dadurch gekennzeichnet, daß** der Teil (124) des Klappenelementes (12) zwischen einer gedachten Achsenlinie (240) zwischen den Aussparungen (122, 123) und einer herzwärts gerichteten Spitze (125) des Klappenelementes (12) gegenüber dem auf der anderen Seite der Achslinie (240) gelegenen anderen Teil (126) des Klappenelementes (12) gewinkelt ausgebildet ist.

10. Prothese nach einem oder mehreren der Ansprüche 1 bis 9, **dadurch gekennzeichnet, daß** drei Klappenelemente (12, 120, 121) vorgesehen sind.

11. Prothese nach Anspruch 10, **dadurch gekennzeichnet, daß** durch geeignete Anordnung und Größe der Klappenelemente (12, 120, 121) vier in bezug auf den Strömungsquerschnitt im wesentlichen gleichgroße Durchflußöffnungen (150, 151, 152, 153) in der Innenöffnung (15) des Ringkörpers (11) gebildet werden.

12. Prothese nach einem oder mehrere der Ansprüche 1 bis 11, **dadurch gekennzeichnet, daß** die Innenöffnung (15) des Ringkörpers (11) von der zur Aorta (13) gerichteten Ausflußöffnung (17) des Blutes durch die Innenöffnung (15) des Ringkörpers (11) hindurch zu seiner Einflußöffnung (16) im Querschnitt sich zunächst im wesentlichen bis zur Ebene der Vorsprünge (19, 190, 191) verengend und im wesentlichen der Ebene der Vorsprünge (19, 190, 191) nachfolgend stark gekrümmt erweiternd ausgebildet ist.

13. Prothese nach einem oder mehreren der Ansprüche 1 bis 12, **dadurch gekennzeichnet, daß** sich der Außenquerschnitt des Ringkörpers (11) von der Ausflußöffnung (17) im wesentlichen bis zur Ebene der Vorsprünge (19, 190; 191) vergrößert.

14. Prothese nach einem oder mehreren der Ansprüche 1 bis 13, **dadurch gekennzeichnet, daß** der Ringkörper (11) an seiner Außenwandung (115) im wesentlichen zwei voneinander beabstandete, nutartige, in den Ringkörper (11) herumlaufende Vertiefungen (112, 113) aufweist.

15. Prothese nach Anspruch 14, **dadurch gekennzeichnet, daß** in einer Vertiefung (112) ein um den Ringkörper (11) herumlaufender Nahtring (114) angeordnet ist.

16. Prothese nach einem oder mehreren der Ansprüche 1 bis 14, **dadurch gekennzeichnet, daß** der Ringkörper (11) und/oder die Klappenelemente (12; 120; 121) aus Titan oder einer Titanlegierung bestehen.

17. Prothese nach einem oder mehreren der Ansprüche 1 bis 16, **dadurch gekennzeichnet, daß** der Ringkörper (11) und/oder die Klappenelemente (12; 120; 121) mit einer Hartstoffschicht beschichtet sind.

## Claims

1. Prosthesis (10) for the replacement of the aorta and mitral valve of the heart (14), comprising an annular body (11), accommodating in jointed fashion a plurality of valve elements (12, 120, 121), which can be introduced into the aorta (13) and/or into the mitral valve ring, and can be secured there by surgical means, whereby the valve elements (12, 120, 121), depending on the pivot position, either clear or block the passage through the inner aperture (15) of the annular body (11), **characterised in that** the joints (18, 180, 181) of the valve elements (12, 120, 121) are formed on the ring side at projections (19, 190, 191) which extend into the inner aperture (15) of the annular body (11).

2. Prosthesis according to Claim 1, **characterised in that** at least one pair of webs (21, 22), extending essentially into the inner aperture (15) of the annular body (11) and spaced at a distance from one another in the direction (25) of the blood flow through the annular body (11), are formed in the area of their ends (20; 200; 201) facing away from the annular body (11), said webs in each case forming a ring-side joint (23, 230, 231).

3. Prosthesis according to one or both of Claims 1 or 2, **characterised in that** the joints (23; 230; 231) of the valve elements (12; 120; 121) are formed on the valve side by two flat cut-out elements (122, 123), essentially symmetrical to an imaginary mid-line (24) of the essentially flat-formed valve elements (12; 120; 121).

4. Prosthesis according to one or more of Claims 1 to 3, **characterised in that** at least one of the webs (21; 22) is formed as integral to the valve elements (12; 120; 121) adjacent in each case to a projection (19; 190; 191).

5. Prosthesis according to one or both of Claims 3 or 4, **characterised in that** the valve element (12; 120; 121) is designed as jointed in the area of the cut-out elements (122, 123) between the webs (21, 22) spaced at a distance from one another of the projections (19; 190; 191) corresponding on both sides to the valve element (12; 120; 121) at the annular body (11).

6. Prosthesis according to one or more of Claims 2 to 5, **characterised in that** the web (21) of the web pair which faces the aorta has a cambered joint surface (210) running essentially orthogonally to the direction (25) of the blood flow through the annular body (11), on which surface the valve element (12; 120; 121) rolls during its opening and closing movement.

7. Prosthesis according to one or more of Claims 1 to 6, **characterised in that** the web (22) of the web pair which faces away from the aorta has two contact surfaces (232; 233) running essentially orthogonally to the direction (25) of the blood flow through the annular body (11), which delimit the opening position (26) and the closed position (27) respectively of the valve element (12).

8. Prosthesis according to one or more of Claims 1 to 7, **characterised in that** the valve element (12; 120; 121) is designed as spherical.

9. Prosthesis according to one or more of Claims 1 to 8, **characterised in that** the part (124) of the valve element (12) between an imaginary axis line (240) between the cut-out elements (122, 123) and a tip (125) of the valve element (12), pointing in the direction of the heart, is formed at an angle in relation to the other part (126) of the valve element (12) located on the other side of the axis line (240).

10. Prosthesis according to one or more of Claims 1 to 9, **characterised in that** three valve elements (12, 120 121) are provided for.

11. Prosthesis according to Claim 10, **characterised in that**, by a suitable arrangement and size of the valve elements (12, 120, 121), four throughflow apertures (150, 51, 152, 153), of essentially the same size with regard to the flow cross-section, are formed in the inner aperture (15) of the annular body (11).

12. Prosthesis according to one or more of Claims 1 to 11, **characterised in that** the inner aperture (15) of the annular body (11) is designed such that in cross-section it initially narrows from the outflow aperture (17) of the blood pointing to the aorta (13), through the inner aperture (15) of the annular body (11) to its inflow aperture (16) as far as the plane of the projections (19, 190, 191), and thereafter widens, substantially cambered, essentially in the plane of the projections (19, 190, 191).

13. Prosthesis according to one or more of Claims 1 to 12, **characterised in that** the outer cross-section of the annular body (11) increases from the outflow aperture (17) essentially as far as the plane of the projections (19, 190; 191).

14. Prosthesis according to one or more of Claims 1 to 13, **characterised in that** the annular body (11) has on its outer wall (115) essentially two indentations (112, 113) spaced at a distance from one another, in groove fashion, running around in the annular body (11).

15. Prosthesis according to Claim 14, **characterised in that** arranged in one indentation (112) is a suture ring (114), running around the annular body (11).

16. Prosthesis according to one or more of Claims 1 to 14, **characterised in that** the annular body (11) and/or the valve elements (12; 120; 121) are made of titanium or a titanium alloy.

17. Prosthesis according to one or more of Claims 1 to 16, **characterised in that** the annular body (11) and/or the valve elements (12; 120; 121) are coated with a layer of resistant material.

## Revendications

1. Prothèse (10), pour le remplacement de la valvule aortique et/ou de la valvule mitrale du coeur (14), comprenant un corps annulaire (11) recevant, de manière articulé, une pluralité d'éléments de valvule (12, 120, 121), ledit corps annulaire (11) étant susceptible d'être introduit dans l'aorte (13) et/ou l'anneau de valvule mitrale et d'y être fixé à l'aide de dispositions chirurgicales, les éléments de valvule (12, 120, 121), selon la position en pivotement, libérant ou bloquant le passage à travers l'ouverture intérieure (15) du corps annulaire (11), **caractérisé en ce que** les articulations (18, 180, 181) des éléments de valvule (12, 120, 121) sont réalisées, côté anneau, sur des saillies (19, 190, 191) s'étendant dans l'ouverture intérieure (15) du corps annulaire (11).

2. Prothèse selon la revendication 1, **caractérisée en ce que**, sur les saillies (19, 190, 191), dans la zone de leurs extrémités (20 ; 200 ; 201) opposées au corps annulaire (11), sont réalisées au moins une paire de nervures (21, 22), s'étendant sensiblement dans l'ouverture intérieure (15) du corps annulaire (11), espacées les unes des autres dans la direction d'écoulement (25) du sang à travers le corps annulaire (11), nervures formant chacune une articulation (23, 230, 231) située côté anneau.

3. Prothèse selon l'une ou les deux des revendications 1 ou 2, **caractérisée en ce que** les articulations (23, 230, 231) des éléments de valvule (12 ; 120 ; 121) sont formées, côté valvule, par deux évidements (122, 123) plats, sensiblement symétriquement à un axe médian (24) imaginaire des éléments de valvule (12 ; 120 ; 121) sensiblement plats.

4. Prothèse selon l'une ou plusieurs des revendications 1 à 3, **caractérisée en ce qu'**au moins l'une des nervures (21 ; 22) est réalisée d'une seul pièce pour des éléments de valvule (12 ; 120 ; 121) chaque fois voisins sur une saillie (19 ; 190 ; 191).

5. Prothèse selon l'une ou les deux des revendications 3 ou 4, **caractérisée en ce que** l'élément de valvule (12 ; 120 ; 121) est saisi, de manière articulé, dans la zone des évidements (122, 123), entre les nervures (21, 22), espacées les unes des autres, des saillies (19 ; 190 ; 191) correspondant, de part et d'autre, à l'élément de valvule (12 ; 120 ; 121) sur le corps annulaire (11):

6. Prothèse selon l'une ou plusieurs des revendications 2 à 5, **caractérisée en ce que** la nervure (21), tournée vers l'aorte, de la paire de nervures présente, s'étendant sensiblement perpendiculairement à la direction d'écoulement (25) du sang à travers le corps annulaire (11), une surface d'articulation (210) incurvée, sur laquelle l'élément de valvule (12 ; 120 ; 121) roule, lors de son mouvement d'ouverture et de fermeture.

7. Prothèse selon l'une ou plusieurs des revendications 1 à 6, **caractérisé en ce que** la nervure (22), opposée à l'aorte, de la paire de nervures présente, s'étendant sensiblement perpendiculairement à la direction d'écoulement (25) du sang à travers le corps annulaire (11), deux surfaces de buté (232 ; 233) incurvée, délimitant chacune, de manière définie, la position d'ouverture (26) et la position de fermeture (27) de l'élément de valvule (12).

8. Prothèse selon l'une ou plusieurs des revendications 1 à 7, **caractérisé en ce que** l'élément de valvule (12 ; 120 ; 121) est de configuration sphérique.

9. Prothèse selon l'une ou plusieurs des revendications 1 à 8, **caractérisé en ce que** la partie (124) de l'élément de valvule (12) est de configuration coudée, entre une ligne d'axe (240) imaginaire, entre les évidements (122, 123), et une pointe (125), orientée vers le coeur, de l'élément de valvule (12), par rapport à l'autre partie (126), située sur l'autre côté de la ligne d'axe (240), de l'élément de valvule (12).

10. Prothèse selon l'une ou plusieurs des revendications 1 à 9, **caractérisé en ce que** trois éléments de valvule (12 ; 120 ; 121) sont prévus.

11. Prothèse selon la revendication 10, **caractérisée en ce que**, grâce à un agencement et une taille appropriés des éléments de valvule (12 ; 120 ; 121), quatre ouvertures de passage d'écoulement (150, 151, 152, 153), de grandeur sensiblement identique quant à la section transversale offerte à l'écoulement, sont formées dans l'ouverture intérieure (15) du corps annulaire (11).

12. Prothèse selon l'une ou plusieurs des revendications 1 à 11, **caractérisé en ce que** l'ouverture intérieure (15) du corps annulaire (11), de l'ouverture de sortie d'écoulement (17), tournée vers l'aorte (13), du sang, en passant par l'ouverture intérieure (15) du corps annulaire (11), vers son ouverture d'entrée d'écoulement (16), présente une section transversale qui va d'abord en rétrécissant, sensiblement jusqu'au plan des saillies (19, 190, 191) et va en s'élargissant ensuite de manière fortement incurvée sensiblement dans le plan des saillies (19, 190, 191).

13. Prothèse selon l'une ou plusieurs des revendications 1 à 12, **caractérisé en ce que** la section transversale extérieure du corps annulaire (11) va en augmentant, en évoluant de l'ouverture de sortie d'écoulement (17), sensiblement jusqu'au plan des saillies (19, 190, 191).

14. Prothèse selon l'une ou plusieurs des revendications 1 à 13, **caractérisé en ce que** le corps annulaire (11) présente, sur sa paroi extérieure (115), essentiellement deux cavités (112, 113) espacées l'une de l'autre, du genre de gorges, faisant le pourtour dans le corps annulaire (11).

15. Prothèse selon la revendication 14, **caractérisée en ce qu'**un anneau à suturer (114), faisant le pourtour du corps annulaire (11), est disposé dans une cavité (112).

16. Prothèse selon l'une ou plusieurs des revendications 1 à 14, **caractérisé en ce que** le corps annulaire (11) et/ou les éléments de valvule (12 ; 120 ; 121) est/sont composé(s) de titane ou d'un alliage de titane.

17. Prothèse selon l'une ou plusieurs des revendications 1 à 16, **caractérisé en ce que** le corps annulaire (11) et/ou les éléments de valvule (12 ; 120 ; 121) est/sont revêtus(s) d'une couche de matériau dur.
